(19) Europäisches Patentamt
European Patent Office
Office européen des brevets

(11) **EP 4 782 831 A1**

(12) **EUROPEAN PATENT APPLICATION**
published in accordance with Art. 153(4) EPC

(43) Date of publication:
**29.07.2026  Bulletin 2026/31**

(21) Application number: **24868301.3**

(22) Date of filing: **19.09.2024**

(51) International Patent Classification (IPC):
*G01N 33/20* (2019.01)

(52) Cooperative Patent Classification (CPC):
**G01N 33/20**

(86) International application number:
**PCT/JP2024/033405**

(87) International publication number:
**WO 2025/063224 (27.03.2025 Gazette 2025/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **21.09.2023  JP 2023156362**

(71) Applicant: **Proterial, Ltd.
Tokyo 135-0061 (JP)**

(72) Inventor: **FUKUMOTO, Shiho
Tokyo 135-0061 (JP)**

(74) Representative: **Cabinet Beaumont
4, Place Robert Schuman
B.P. 1529
38025 Grenoble Cedex 1 (FR)**

(54)    **STEEL EVALUATION METHOD AND PROGRAM**

(57)    Provided is a method for evaluating steel capable of obtaining steel having stable quality by using a new variable for evaluating dimensional change characteristics.

A method for evaluating steel includes acquiring a forging ratio when a steel ingot (21) is forged to obtain a forged material (22), acquiring dimensions of a rectangular parallelepiped-shaped sample (40), for the forged material (22), the sample having one side in an extension direction during forging, and calculating a variable related to isotropy of the sample (40) based on the forging ratio and the dimensions. The variable is an isotropy variable. Dimensional change characteristics of samples (40) respectively taken from a plurality of forged materials (22) are actually measured, the isotropy variable is calculated for each sample (40), and a graph using the isotropy variable on a first axis and the dimensional change characteristics on a second axis is output based on the isotropy variable and the dimensional change characteristics.

FIG.1

EP 4 782 831 A1

## Description

Technical Field

The present invention relates to a method for evaluating steel, and program.

Background Art

Steel used for bending, drawing, and blanking of sheet materials is generally manufactured by performing hot working such as forging or rolling, annealing, machining, and quenching and tempering treatments on a cast steel ingot. A known issue that arises during a quenching and tempering process is dimensional change, and when the dimensional change is large, it leads to an increase in the number of processing steps after heat treatment, and thus various studies have conventionally been conducted to suppress dimensional change.

For example, in Patent Literature 1, in order to reduce heat-treatment-induced dimensional change occurring after quenching and tempering, the applicant of this application found that such heat-treatment-induced dimensional change can also be suppressed by reducing segregation, and proposes a cold die steel capable of reducing segregation and suppressing dimensional change by adopting a "segregation index K" obtained by combining the solid-phase/liquid-phase concentration partition coefficients of respective elements and changes in specific gravity, and by optimally adjusting its value.

Further, Patent Literature 2 describes a cold die steel having excellent dimensional change control characteristics, which is obtained by reducing primary carbides remaining in an as-cast (as-cast without further processing) state for the purpose of suppressing dimensional change, and by adding appropriate amounts of Ni, Al, and Cu based on a component composition that can suppress occurrence of dimensional change as much as possible within a range of satisfying various properties. According to the invention of Patent Literature 1, Ni and Al form intermetallic compounds and precipitate during tempering (aging) in a secondary hardening region of tool steel, thereby acting on dimensional change in a shrinkage direction, so that expansion during heat treatment can be offset.

Prior Art Documents

Patent Literature:

Patent Literature 1: Japanese Patent Application Laid-Open No. 2006-152356
Patent Literature 2: Japanese Patent Application Laid-Open No. 2006-169624

Non-Patent Literature:

Non-Patent Literature 1: G.A.Roberts, Tool Steel 5th(1998), p338
Non-Patent Literature 2: F.Rapatz, Die Edelstahl(1962), p945
Non Patent Literature 3: "Influence of Coarse Carbide Volume on Anisotropy of Dimensional Change at Heat Treatment of Cold Work Die steels", Takayuki Shimizu and Koichiro Inoue, Electric Steelmaking, 2007, 78.4: 289

Summary of the Invention

Problems to be Solved by the Invention

On the other hand, according to experimental results obtained by the applicant of this application, there are cases in which dimensional change characteristics vary even with the same alloy composition. When a mold has a rectangular parallelepiped shape, such variation is expressed as anisotropy, meaning that rates of dimensional change after quenching and tempering, relative to the dimensions before quenching, differ among the thickness, width, and length dimensions. To date, as metallurgical technical literature related to such anisotropy of dimensional change, there have been two reported examples regarding observed dimensional change phenomena (Non-Patent Literatures 1 and 2). Further, according to research examples on cold work tool steel (cold die steel), it has been reported that such dimensional change is unlikely to occur when crystallized carbides are absent in the cold die steel, and that anisotropy occurs when crystallized carbides are present, based on FEM analysis taking into account a distribution of crystallized carbides (Non-Patent Literature 3). Accordingly, stabilization of quality when evaluated based on dimensional change has also been an issue. Further, even though the dimensional change can be reduced by eliminating component segregation as described in Patent Literature 1, a degree of influence on dimensional change differs among elements, and it has taken time to determine which elemental species should be focused on to improve segregation.

Accordingly, an object of the invention is to provide an evaluation method that makes it possible to obtain steel having stable quality by using a new variable for evaluating dimensional change characteristics.

Means for Solving the Problem

A method for evaluating steel includes acquiring a forging ratio when a steel ingot is forged to obtain a forged material, acquiring dimensions of a rectangular parallelepiped-shaped sample, for the forged material, the sample having one side in an extension direction during forging, and calculating a variable related to isotropy of the sample based on the forging ratio and the dimensions.

Advantageous Effects of the Invention

According to the invention, it is possible to provide an evaluation method for obtaining steel having stable quality.

Brief Description of the Drawings

FIG. 1 is an explanatory diagram illustrating an overview of a method for evaluating steel.
FIG. 2 is an explanatory diagram illustrating changes in unit cells before and after forging.
FIG. 3 is an explanatory diagram illustrating a configuration of an information processing apparatus.
FIG. 4 is a graph illustrating the heat-treatment-induced dimensional change of specimen number 1-L.
FIG. 5 is a graph illustrating the heat-treatment-induced dimensional change of specimen number 1-S.
FIG. 6 is a graph illustrating the heat-treatment-induced dimensional change of specimen number 2-L.
FIG. 7 is a graph illustrating the heat-treatment-induced dimensional change of specimen number 2-S.
FIG. 8 is a graph illustrating the heat-treatment-induced dimensional change of specimen number 3-L.
FIG. 9 is a graph illustrating the heat-treatment-induced dimensional change of specimen number 4-L.
FIG. 10 is a graph illustrating a relationship between an isotropy variable and a sample standard deviation s of dimensional-change rates.
FIG. 11 is an example of an Ms point mapping image obtained by imaging mapping data of an Ms point.
FIG. 12 is an example of a binarized image obtained by performing normalization processing and binarization processing on FIG. 11.
FIG. 13 is a diagram in which principal component vectors obtained by principal component analysis are superimposed on FIG. 11.
FIG. 14 is a graph illustrating a relationship between the isotropy variable and a first principal component contribution ratio.
FIG. 15 is a flowchart for describing a processing flow of a program for calculating the isotropy variable.

Description of the Invention

[Embodiment 1]

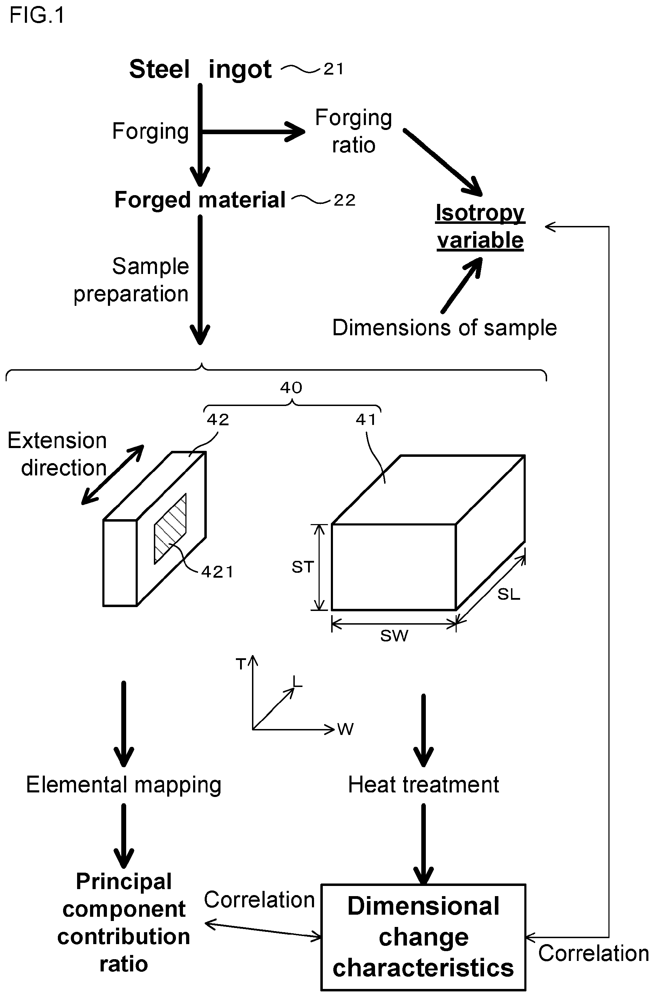

FIG. 1 is an explanatory diagram illustrating an overview of a method for evaluating steel. By adjusting a composition by mixing various alloying elements into iron and performing casting, a steel ingot 21 is produced. The steel ingot 21 is forged to obtain a forged material 22 having a dense and tough **microstructure**. In a forging process, hot forging, cooling, and annealing are repeated a plurality of times. The forged material 22 subjected to annealing but not subjected to quenching and tempering may be referred to as an annealed material, and a forged material subjected to quenching and tempering may be referred to as a quenched-and-tempered material.

A degree of forging is represented by a forging ratio. The forging ratio is also referred to as a forging reduction ratio, and indicates a ratio of cross-sectional areas before and after forging. Details of the forging ratio are defined in Japanese Industrial Standards (JIS), namely JIS G0701-1957, "Method of Indicating Forging Reduction Ratio in Forging Operations of Steel Materials".

A sample 40 is prepared from the forged material 22. The sample 40 includes a dimensional-change measurement sample 41 and a mapping sample 42. The dimensional-change measurement sample 41 is used for actual measurement of dimensional change characteristics. The mapping sample 42 is used for calculating a principal component contribution ratio. Methods of obtaining the dimensional change characteristics and the principal component contribution ratio will be described later.

Studies conducted by the applicant of this application have revealed a correlation between the dimensional change characteristics and the principal component contribution ratio. Accordingly, the dimensional change characteristics can be evaluated by calculating the principal component contribution ratio. Calculation of the principal component contribution ratio is easier to automate and less likely to result in data variation than actual measurement of the dimensional change characteristics. Therefore, the principal component contribution ratio can be used as a surrogate characteristic for the dimensional change characteristics.

However, to calculate the principal component contribution ratio, it is necessary to perform elemental mapping using an FE-EPMA (Field Emission - Electron Probe Micro Analyzer), as described later. Since the FE-EPMA is expensive equipment that can be used for various measurements, using the FE-EPMA entails significant costs.

Accordingly, the applicant of this application has developed a method for evaluating steel in which an isotropy variable Q, calculated based on a forging ratio and dimensions of the sample 40, is used as a surrogate characteristic for the dimensional change characteristics. A method of calculating the isotropy variable Q will be described later. The isotropy variable is an example of a variable related to isotropy of the sample 40.

With reference to FIG. 1, orientations when collecting each sample 40 will be described. In the following description, an extension direction during forging is referred to as an L direction. Two directions perpendicular to the L direction are referred to as a W direction and a T direction. The W direction is perpendicular to the T direction. That is, the L direction, the W direction, and the T direction form an orthogonal coordinate system having three mutually perpendicular axes.

The dimensional-change measurement sample 41 is a rectangular parallelepiped taken from the forged material 22, having sides respectively parallel to the L direction, the W direction, and the T direction. That is, the dimensional-change measurement sample 41 has a rectangular parallelepiped shape with a first side oriented in the extension direction during forging. Lengths of the sides of the dimensional-change measurement sample 41 in the L direction, the W direction, and the T direction in a state cooled to room temperature are denoted by symbols SL, SW, and ST, respectively.

For the dimensional-change measurement sample 41, actual measurement of dimensions before heat treatment, heat treatment such as quenching and tempering, and actual measurement of dimensions after heat treatment are sequentially performed. Dimensional change characteristics are calculated from dimensional changes before and after the heat treatment. Specific examples of the dimensional change characteristics will be described later.

The mapping sample 42 is a flat plate taken from the forged material 22 and perpendicular to the W direction. The mapping sample 42 may be a rectangular parallelepiped having faces respectively perpendicular to the L direction, the W direction, and the T direction.

In the following description, a plane perpendicular to the W direction is referred to as a T-L plane. One surface of the mapping sample 42 is smoothly polished to form a mapping region 421 suitable for elemental mapping using the FE-EPMA. In FIG. 1, the mapping region 421 is indicated by hatching.

Using an elemental mapping function of the FE-EPMA, a matrix composition of the mapping region 421 is mapped. A mapping result is analyzed to calculate a principal component contribution ratio. A method of calculating the principal component contribution ratio will be described later. Note that, in the present embodiment, a principal component contribution ratio of the T-L plane is described. However, the principal component contribution ratio can be similarly obtained for a T-W plane perpendicular to the L direction and for an L-W plane perpendicular to the T direction.

[Method of calculating isotropy variable Q]

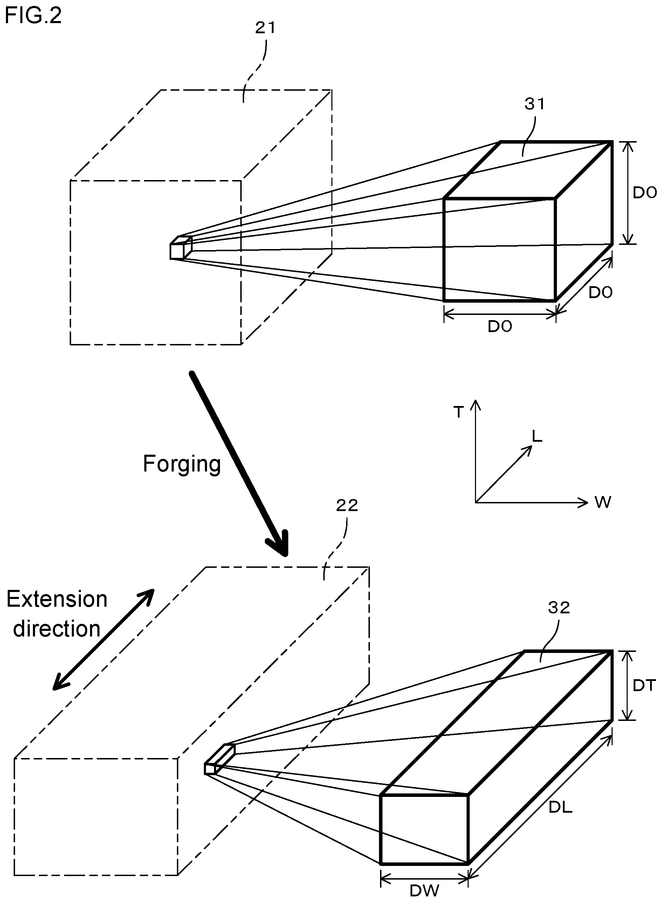

FIG. 2 is an explanatory diagram illustrating changes in unit cells before and after forging. With reference to FIG. 1 and FIG. 2, an equation for calculating the isotropy variable Q will be described. Note that, since the shapes of the steel ingot 21 and the forged material 22 are not necessarily rectangular parallelepipeds, outer contour lines of the steel ingot 21 and the forged material 22 are illustrated by imaginary lines.

In the following description, a minute cube virtually defined inside the steel ingot 21 is referred to as an as-cast unit cell 31. Each side of the as-cast unit cell 31 is parallel to one of the L direction, the W direction, and the T direction. A length of one side of the as-cast unit cell 31 is denoted by D0.

The as-cast unit cell 31 includes iron and trace alloying elements added for composition adjustment. In the as-cast unit cell 31, segregation occurs in which iron atoms and atoms of the trace alloying elements are unevenly distributed. A degree of segregation differs among the L direction, the W direction, and the T direction.

As described above, the forged material 22 is produced by forging the steel ingot 21. In the following description, a forging ratio is denoted by FR. The as-cast unit cell 31 inside the steel ingot 21 is deformed into a rectangular parallelepiped by forging, thereby becoming a post-forging unit cell 32. Each side of the post-forging unit cell 32 is parallel to one of the L direction, the W direction, and the T direction. Lengths of sides of the post-forging unit cell 32 in the L direction, the W direction, and the T direction are denoted by DL, DW, and DT, respectively.

Since the volume of the unit cell remains unchanged before and after forging, Equation (1) is satisfied.

$$D0 \cdot D0 \cdot D0 = DL \cdot DW \cdot DT \quad (1)$$

The definition of the forging ratio FR is given by Equation (2).

[Formula 1]

$$FR = \frac{D0^2}{DT \cdot DW} \quad \cdots \cdots \quad (2)$$

Assuming that the dimensional-change measurement sample 41 taken from the forged material 22 is constituted by post-forging unit cells 32 of identical dimensions arranged without any gaps, the numbers of the post-forging unit cells 32 arranged along the sides of the dimensional-change measurement sample 41 in the L direction, the W direction, and the T direction are denoted by NL, NW, and NT, respectively. Definitions of NL, NW, and NT are given by Equations (2) to (4).

$$NL = SL/DL \quad (2)$$

$$NW = SW/DW \quad (3)$$

$$NT = ST/DT \quad (4)$$

The isotropy variable Q is defined by Equation (5). From Equation (5), the isotropy variable Q is a dimensionless quantity having no unit.

[Formula 2]

$$Q = \frac{NL}{NT} \cdot \frac{NL}{NW} \quad \cdots \cdots \quad (5)$$

Since NL, NW, and NT cannot be directly measured, the isotropy variable Q cannot be directly obtained from Equation (5). However, by substituting Equations (1) to (4) into Equation (5), Equation (6) is obtained.

[Formula 3]

$$Q = \frac{SL^2}{(ST \cdot SW) \cdot FR^3} \quad \cdots \cdots \quad (6)$$

From Equation (6), the isotropy variable Q can be calculated from SL, SW, and ST, which are the lengths of the three sides of the dimensional-change measurement sample 41 prepared from the forged material 22, and the forging ratio FR. Note that, in calculating the isotropy variable Q using Equation (6), it is not necessary to actually prepare the dimensional-change measurement sample 41, and it is sufficient to virtually define the dimensions of the dimensional-change measurement sample 41.

Accordingly, the isotropy variable Q can be calculated far more easily than the dimensional change characteristics calculated from dimensional values of the dimensional-change measurement sample 41 actually measured before and after heat treatment, and the principal component contribution ratio calculated through elemental mapping and the like of the mapping sample 42. The fact that the isotropy variable Q has a correlation with the dimensional change characteristics and can be used as a surrogate characteristic for the dimensional change characteristics will be described in an experimental example below.

[Overview of method of measuring and calculating dimensional change characteristics]

As described above, the dimensional-change measurement sample 41 is taken from the forged material 22. After the dimensional-change measurement sample 41 is cooled to room temperature, lengths of respective sides before heat treatment are measured. After completion of heat treatment on the dimensional-change measurement sample 41, lengths of respective sides of the mapping sample 42 cooled to room temperature are measured.

For each of the L direction, the W direction, and the T direction of the dimensional-change measurement sample 41, a dimensional change rate before and after heat treatment is calculated by Equation (7). For one dimensional-change measurement sample 41, dimensional change rates in a total of three directions, namely, the L direction, the W direction, and the T direction, are calculated.

[Formula 4]

$$\text{Dimensional change rate} = \frac{LB - LA}{LA} \quad \cdots\cdots (7)$$

LA is a length before heat treatment.
LB is a length after heat treatment.

From the dimensional change rates in the three directions, an average value and a sample standard deviation are calculated. Dimensional change rates in the L direction, the W direction, and the T direction, and the average value and the sample standard deviation of the dimensional change rates in the three directions are used as the dimensional change characteristics.

[Overview of method of calculating principal component contribution ratio]

As described above, the dimensional-change measurement sample 41 is prepared from the forged material 22. One surface of the mapping sample 42 is smoothly polished to form the mapping region 421.

Using the elemental mapping function of the FE-EPMA, mapping data related to the matrix composition of the mapping region 421 is obtained. Measurement conditions of the FE-EPMA are, for example, a beam diameter of 20 $\mu$m, an accelerating voltage of 15 kV, and a probe current of 0.1 $\mu$A for a measurement field of view of 8 mm × 4 mm.

From the mapping data related to the matrix composition, mapping data of an Ms point is generated. The Ms point is a temperature at which transformation from austenite, which is a crystal structure of steel, to martensite starts.

To generate the Ms-point mapping data, for example, it is possible to use a calculation method based on Ms-point prediction equation by Ishida (ISHIDA, K., "Calculation of the Effect of Alloying Elements on the Ms Temperature in Steels", Journal of Alloys and Compounds, 1995, Vol. 220, pp. 126-131).

Based on the Ms-point mapping data, a mapping image can be generated. Binarization processing is performed on the mapping image using known image processing software or the like. For example, a discriminant analysis method is used to determine a threshold value for binarization.

Next, principal component analysis is performed on the binarized Ms-point mapping image, and a first principal component and a second principal component at each measurement location are calculated. The first principal component is a principal component vector corresponding to the largest eigenvalue, and the second principal component is a principal component vector corresponding to the second largest eigenvalue. For example, data at three measurement locations are extracted in descending order of the magnitude of the first principal component. At each measurement location, a contribution ratio of the first principal component and a contribution ratio of the second principal component are calculated by Equations (8) and (9).

[Formula 5]

$$\text{Contribution ratio of the first principal component} = \frac{a}{a+b}$$
$$\cdots\cdots (8)$$

$$\text{Contribution ratio of the second principal component} = \frac{b}{a+b}$$
$$\cdots\cdots (9)$$

a is a magnitude of the first principal component.

b is a magnitude of the second principal component.

The significance of the first principal component and the second principal component will be briefly described. When a ratio of the first principal component to the second principal component is close to 1, each of the contribution ratios of the first principal component and the second principal component is approximately 0.5. This indicates that the difference in Ms point between a measurement point and surrounding points thereof is small and that a distribution of the Ms point is isotropic. Accordingly, steel materials in which the contribution ratios of the first principal component and the second principal component are close to 0.5 are likely to exhibit little dimensional change due to heat treatment.

As the ratio of the first principal component to the second principal component deviates further from 1, the difference in Ms point between the measurement point and surrounding points thereof becomes larger, indicating that the distribution of the Ms point is anisotropic. Accordingly, steel materials in which the contribution ratios of the first principal component and the second principal component deviate from 0.5 are likely to exhibit dimensional change due to heat treatment.

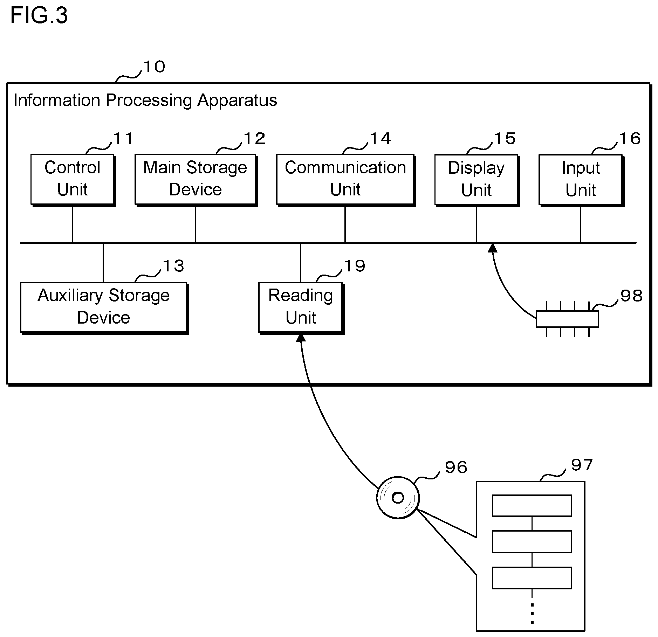

FIG. 3 is an explanatory diagram illustrating a configuration of an information processing apparatus 10. The information processing apparatus 10 includes a control unit 11, a main storage device 12, an auxiliary storage device 13, a communication unit 14, a display unit 15, an input unit 16, a reading unit 19, and a bus.

The control unit 11 is an arithmetic control device that executes a program according to the present embodiment. As the control unit 11, one or more CPUs (Central Processing Units), GPUs (Graphics Processing Units), multi-core CPUs or the like are used. The control unit 11 is connected to each of the hardware components constituting the information processing apparatus 10 via the bus.

The main storage device 12 is a storage device such as an SRAM (Static Random Access Memory), a DRAM (Dynamic Random Access Memory), or a flash memory. The main storage device 12 temporarily stores information required during processing performed by the control unit 11 and a program being executed by the control unit 11.

The auxiliary storage device 13 is a storage device such as an SRAM, a flash memory, a hard disk, or a magnetic tape. The auxiliary storage device 13 stores programs executed by the control unit 11 and various types of data required for execution of the programs.

The communication unit 14 is an interface that performs communication between the information processing apparatus 10 and a network. The display unit 15 is, for example, a liquid crystal display panel or an organic EL (electroluminescence) panel. The input unit 16 is, for example, an input device such as a keyboard, a mouse, or a microphone. The display unit 15 and the input unit 16 may be stacked to form a touch panel.

A portable recording medium 96 is, for example, a USB (Universal Serial Bus) memory, a CD-ROM (Compact Disc Read Only Memory), a magneto-optical disc medium, another optical disc medium, or an SD memory card. The portable recording medium 96 stores a program 97 described later.

For example, the reading unit 19 is an interface, such as a USB connector, a CD-ROM drive, or an SD memory reader, to which the portable recording medium 96 can be connected. The semiconductor memory 98 stores the program 97 and is a memory that can be installed inside the information processing apparatus 10.

The information processing apparatus 10 is a general-purpose personal computer, a tablet, a large-scale computer, a virtual machine operating on a large-scale computer, or a quantum computer. The information processing apparatus 10 may include hardware such as a plurality of personal computers or large-scale computers that perform distributed processing. The information processing apparatus 10 may be configured as cloud computing system. The information processing apparatus 10 may include hardware such as a plurality of personal computers or large-scale computers configured to operate in cooperation with each other.

The program 97 is recorded on the portable recording medium 96. The control unit 11 reads the program 97 via the reading unit 19 and stores the program in the auxiliary storage device 13. In addition, the control unit 11 may read the program 97 stored in the semiconductor memory 98. Furthermore, the control unit 11 may download the program 97 from another server computer (not illustrated) connected via the communication unit 14 and a network (not illustrated), and store the program 97 in the auxiliary storage device 13.

The program 97 is installed as a control program of the information processing apparatus 10 and is loaded into the main storage device 12 and executed. The program 97 according to the present embodiment is an example of a program product.

Experimental Example

The steel ingot 21 was produced. The steel ingot 21 manufactured by a single casting was divided into four steel ingots 21, which were assigned numbers steel ingot No. 1 to steel ingot No. 4. Steel ingot No. 1 and steel ingot No. 3 were taken from a top side of the steel ingot 21 before division. Steel ingot No. 2 and steel ingot No. 4 were taken from a bottom side of the steel ingot 21 before division.

Each steel ingot 21 was subjected to a forging process in which hot forging, cooling, and annealing were repeated, thereby obtaining the forged material 22. The dimensions of the dimensional-change measurement sample 41 taken from each forged material 22 were assumed, and the isotropy variable Q calculated according to Equation (6) is shown in Table 1. A specimen number indicates a number assigned to the assumed dimensional-change measurement sample 41. A first number as the specimen number represents a steel ingot number of the forged material 22, and an alphabet following the hyphen represents an identifier for distinguishing differences in the dimensions of the dimensional-change measurement sample 41.

[Table 1]

| specimen number | steel ingot No. | dimension [ mm ] | | | Isotropy variable Q |
|---|---|---|---|---|---|
| | | SL | SW | ST | |
| 1-L | 1 | 30 | 25 | 20 | 7.670E-03 |
| 1-S | 1 | 10 | 25 | 20 | 8.522E-04 |
| 2-L | 2 | 30 | 25 | 20 | 2.290E-01 |
| 2-S | 2 | 10 | 25 | 20 | 2.545E-02 |
| 3-L | 3 | 30 | 25 | 20 | 2.575E-04 |
| 4-L | 3 | 30 | 25 | 20 | 3.230E-05 |

The fact that the isotropy variable Q shown in Table 2 is correlated with dimensional change characteristics and a principal component contribution ratio, which will be separately described, will be discussed later.

Table 2 shows results of analysis of the component composition of the forged material 22.

[Table 2]

| | | | | | | | | | | | | (mass%) |
|---|---|---|---|---|---|---|---|---|---|---|---|---|
| Sample No. | Sample Cross-Sectional Dimension [mm] | C | Si | Mn | P | S | Ni | Cr | Mo | V | Al | Remainder |
| 1 | 205×475 | 0.66 | 1.48 | 0.75 | 0.022 | 0.054 | 0.09 | 4.91 | 1.48 | 0.15 | 0.067 | Fe and inevitable impurities |
| 2 | 205×475 | 0.60 | 1.47 | 0.75 | 0.021 | 0.050 | 0.08 | 4.86 | 1.43 | 0.15 | 0.069 | Fe and inevitable impurities |
| 3 | 37×425 | 0.65 | 1.37 | 0.81 | 0.025 | 0.053 | 0.10 | 4.89 | 1.43 | 0.14 | 0.061 | Fe and inevitable impurities |
| 4 | 27×375 | 0.64 | 1.37 | 0.80 | 0.025 | 0.053 | 0.10 | 4.91 | 1.40 | 0.14 | 0.060 | Fe and inevitable impurities |

The dimensional-change measurement sample 41 having the dimensions shown in Table 2 was actually collected from each forged material 22. Note that, for each specimen number, six dimensional-change measurement samples 41 were collected from locations adjacent to one forged material 22.

SL, SW, and ST of the dimensional-change measurement sample 41 were measured.

Each dimensional-change measurement sample 41 was subjected to a heat treatment including quenching by semi-cooling for 10 minutes from 1020°C and two tempering treatments. The semi-cooling refers to a time required to reach 510°C from 1020 °C, 510°C being half of 1020°C. The tempering temperature during the tempering treatments was different for each of the six dimensional-change measurement samples 41 assigned the same specimen number.

After completion of the heat treatment, SL, SW, and ST of each dimensional-change measurement sample 41 were measured after returning to room temperature. Using Equation (7), the dimensional change rates in the L direction, T direction, and W direction were calculated for each dimensional-change measurement sample 41. In addition, an average value ave and a sample standard deviation s of the dimensional change rates in the three directions were calculated.

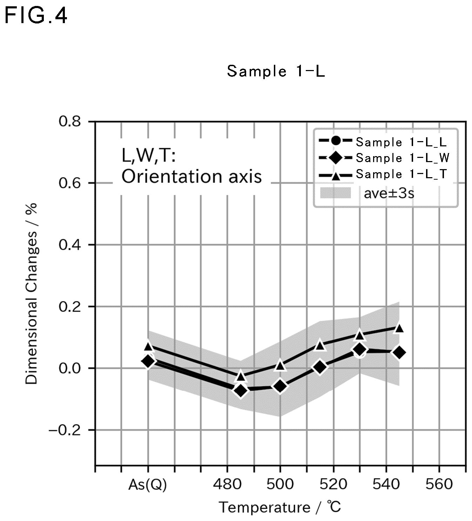

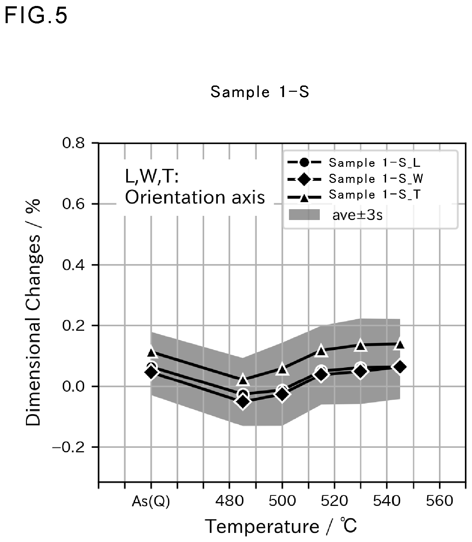

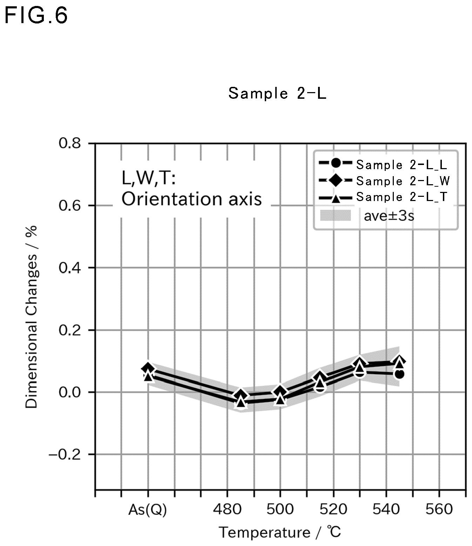

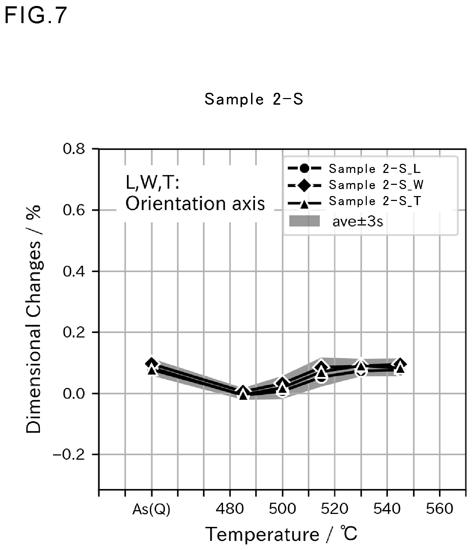

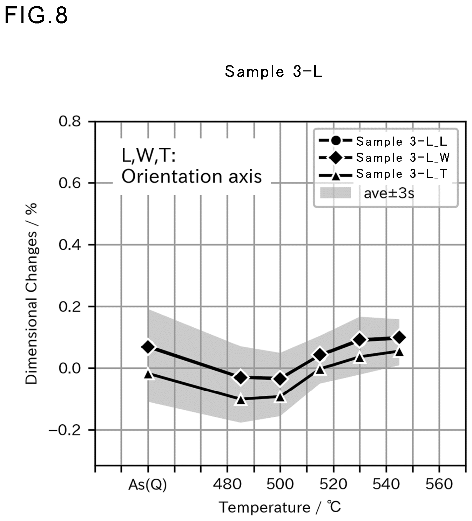

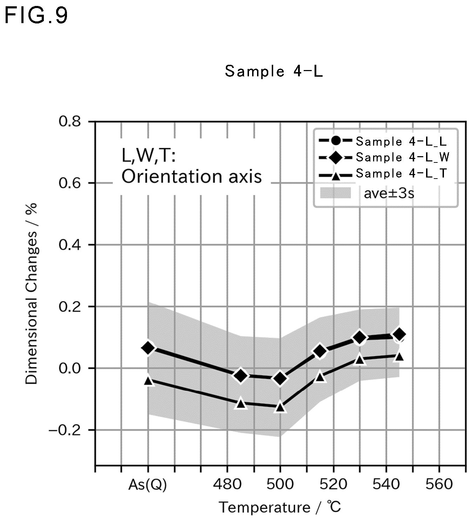

FIG. 4 is a graph illustrating the heat-treatment-induced dimensional change of specimen number 1-L. FIG. 5 is a graph illustrating the heat-treatment-induced dimensional change of specimen number 1-S. FIG. 6 is a graph illustrating the heat-treatment-induced dimensional change of specimen number 2-L. FIG. 7 is a graph illustrating the heat-treatment-induced dimensional change of specimen number 2-S. FIG. 8 is a graph illustrating the heat-treatment-induced dimensional change of specimen number 3-L. FIG. 9 is a graph illustrating the heat-treatment-induced dimensional change of specimen number 4-L.

In the graphs of FIG. 4 to FIG. 9, a horizontal axis indicates a tempering temperature, and a vertical axis indicates the dimensional change rate calculated by Equation (7). "As (Q)" on the horizontal axis indicates that quenching was performed by semi-cooling for 10 minutes from 1020°C. Gray parts indicate ranges calculated as ave $\pm$ 3s for each heat treatment condition.

The dimensional-change rate is indicated as a positive value when the dimension increases after heat treatment, indicating expansion, and as a negative value when the dimension decreases, indicating contraction. A black circle illustrates a measured value of the dimensional-change rate in the L direction. A black square illustrates a dimensional-change rate in the W direction. A black triangle illustrates a dimensional-change rate in the T direction. s is a sample standard deviation calculated from numerical values of three dimensional-change rates in the L, W, and T directions at each tempering temperature.

Table 3 illustrates the isotropy variables Q and the sample standard deviations s when the tempering temperature is 500°C. The isotropy variables Q are identical to the values illustrated in Table 1.

[Table 3]

| Specimen number | Isotropy variable Q | Sample standard deviation s |
|---|---|---|
| 1-L | 7.670E-03 | 0.0407 |
| 1-S | 8.522E-04 | 0.0456 |
| 2-L | 2.290E-01 | 0.0134 |
| 2-S | 2.545E-02 | 0.0127 |
| 3-L | 2.575E-04 | 0.0341 |
| 4-L | 3.230E-05 | 0.0532 |

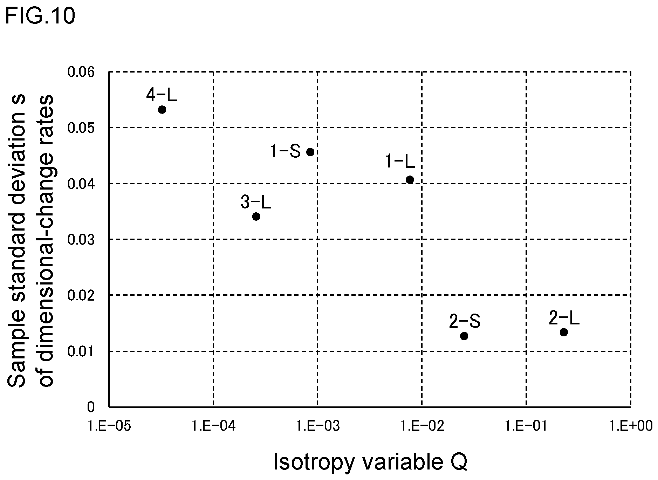

FIG. 10 is a graph illustrating a relationship between the isotropy variable Q and the sample standard deviation s of the dimensional-change rates. The original data of the graph are listed in Table 3. A horizontal axis of FIG. 10 illustrates the isotropy variable Q, and a vertical axis illustrates the standard deviation of the dimensional-change rates. A specimen number is indicated near each data point. FIG. 10 is an example of a graph using the isotropy variable Q on a first axis and the dimensional change characteristics on a second axis.

In FIG. 10, a larger value on the vertical axis indicates a material having a larger difference in dimensional-change rates in the L direction, the W direction, and the T direction, and having stronger anisotropy, and a smaller value on the vertical axis indicates a material having weaker anisotropy.

Comparing FIG. 4 and FIG. 5, it is understood that specimen number 1-S has a larger sample standard deviation s than specimen number 1-L at any tempering temperature. Referring to Table 3, the sample standard deviation s of specimen number 1-S is larger than that of specimen number 1-L by 11 percent or more even though both specimens were taken from the same forged material 22 of steel ingot No. 1. From the above, it is understood that the forged material 22 of steel ingot No. 1 has strong anisotropy.

Similarly, comparing FIG. 6 and FIG. 7, it is understood that both specimen number 2-L and specimen number 2-S exhibit little difference in the sample standard deviation s depending on the tempering temperature. Referring to Table 3, the difference between the sample standard deviation s of specimen number 2-S and the sample standard deviation s of specimen number 2-L is approximately 5 percent. From the above, it is understood that the forged material 22 of steel ingot No. 2 has weak anisotropy.

Referring to Table 3, in steel ingot No. 1 having strong anisotropy, the isotropy variable Q is small for both specimen number 1-L and specimen number 1-S. In steel ingot No. 2 having weak anisotropy, the isotropy variable Q is large for both specimen number 2-L and specimen number 2-S. Referring to FIG. 10, it is understood that there is a negative correlation between the isotropy variable Q and the sample standard deviation s.

From the above, it is understood that there is a correlation between the anisotropy of the forged material 22, which is the variation in dimensional change characteristics depending on direction, and the isotropy variable Q defined by Equation (6), and that the isotropy variable Q can be used as a surrogate characteristic indicating a degree of anisotropy of the forged material 22.

Next, calculation of the principal component contribution ratio will be described. After the dimensional-change measurement sample 41 was taken, the remaining forged material 22 was subjected to a quenching treatment by half cooling for 10 minutes from 1020°C. Then, after the quenching treatment, two tempering treatments at 500°C were performed to prepare a quenched-and-tempered material.

From the quenched-and-tempered material, a mapping sample 42, which is 30 mm in the L direction, 25 mm in the W direction, and 20 mm in the T direction, was taken. One T-L surface was mirror-polished to prepare a mapping region 421.

Using the elemental mapping function of the FE-EPMA, mapping data related to the matrix composition of the mapping region 421 was obtained. The measurement conditions of the FE-EPMA are, for example, a beam diameter of 20 $\mu$m, an accelerating voltage of 15 kV, and a probe current of 0.1 $\mu$A for a measurement field of view of 8 mm $\times$ 4 mm. The mapping region 421 is prepared to be larger than the measurement field of view.

From the mapping data related to the matrix composition, mapping data of the Ms point was prepared. The subsequent processing may be executed by the control unit 11 or may be executed by other hardware.

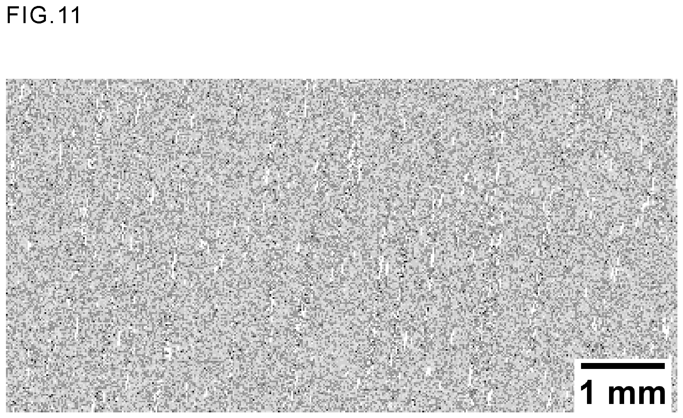

FIG. 11 is an example of an Ms point mapping image obtained by imaging the mapping data of the Ms point. Due to restrictions of the electronic filing software used for the application, the image is monochrome. However, in practice, the Ms point measured at each measurement point is color-mapped, with a low-temperature side illustrated in blue and a high-temperature side illustrated in red. White portions indicate measurement points at which martensitic transformation does not occur and therefore the Ms point cannot be evaluated.

Before performing binarization processing to be described later on the Ms point mapping image, it is preferable to perform normalization processing (equalization processing) using commercially available image processing software such as ImageJ. This normalization processing performs color classification (color reduction conversion) by hierarchy in accordance with a data structure referred to as a lookup table, which divides a distribution of the numerous Ms points into equally spaced hierarchies and is created to improve efficiency by replacing Ms point data with a simple array reference process. As this lookup table, a lookup table incorporating commercially available image processing software may be used.

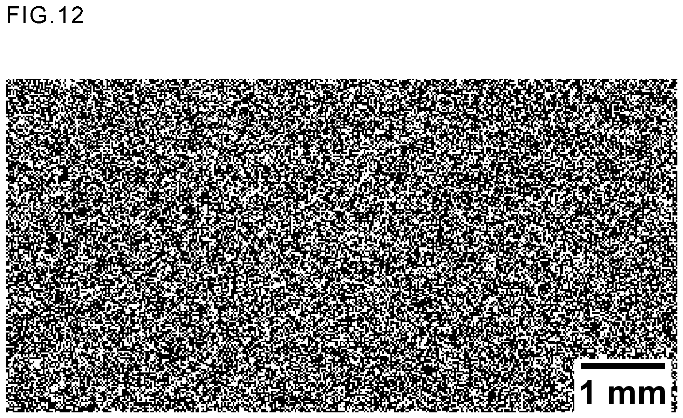

The Ms point mapping image is subjected to binarization processing and converted into a black-and-white image. FIG. 12 is an example of a binarized image obtained by performing the normalization processing and the binarization processing on FIG. 11. A threshold for the binarization processing was determined using a discriminant analysis method (Otsu's method).

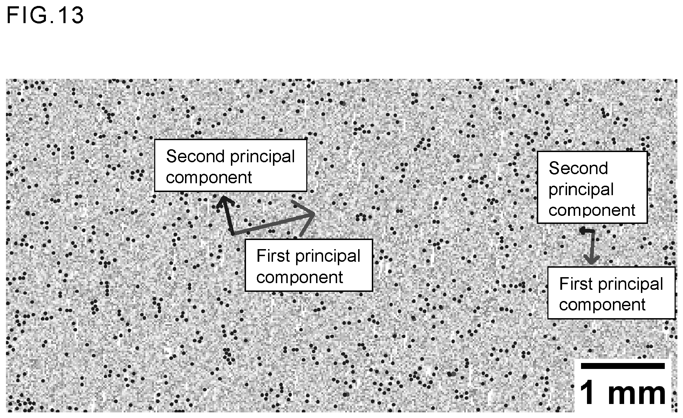

Principal component analysis was performed on the binarized image using a Python-OpenCV library. FIG. 13 is a diagram in which principal component vectors obtained by the principal component analysis are superimposed on FIG. 11. From a single contiguous region in the binarized image, a large arrow indicating the first principal component and a small arrow indicating the second principal component extend. Extremely small arrows are illustrated as points.

Note that, due to restrictions of the electronic filing software used for the application, the image is monochrome. However, in FIG. 13, the first principal component is illustrated by a red arrow and the second principal component is illustrated by a blue arrow in practice.

From one Ms point mapping image, the largest first principal component was extracted. From the magnitude (a) of each first principal component and the magnitude (b) of the second principal component having a common start point, the contribution ratio of the first principal component and the contribution ratio of the second principal component were calculated by Equations (8) and (9). Table 4 illustrates the magnitudes and contribution ratios of the principal components and the isotropy variable Q.

[Table 4]

| Steel ingot No | First principal component | Second principal component | contribution ratio of the first principal component | contribution ratio of the second principal component | isotropy variable Q. |
|---|---|---|---|---|---|
| 1 | 114419 | 36385 | 0.759 | 0.241 | 7.670E-03 |
| 2 | 54657 | 38925 | 0.584 | 0.416 | 2.290E-01 |
| 3 | 98590 | 37140 | 0.726 | 0.274 | 2.575E-04 |
| 4 | 213491 | 39285 | 0.845 | 0.155 | 3.230E-5 |

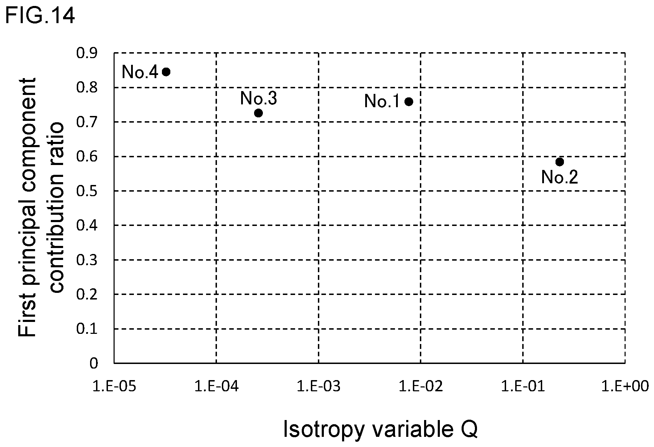

FIG. 14 is a graph illustrating a relationship between the isotropy variable Q and the first principal component contribution ratio. The original data of the graph are shown in Table 4. A horizontal axis of FIG. 14 illustrates the isotropy variable Q. A vertical axis illustrates the first principal component contribution ratio. A steel ingot No. is indicated near each data point.

From Equation (8), the first principal component contribution ratio becomes a value greater than 0.5. As described above, as the first principal component contribution ratio is closer to 0.5, the temperature difference of the Ms point between a measurement point and a surrounding point thereof becomes smaller, indicating that a distribution of the Ms points is isotropic. Accordingly, a steel material having a first principal component contribution ratio close to 0.5 is highly likely to be a steel material in which dimensional change due to heat treatment is unlikely to occur.

Referring to FIG. 14, it is understood that there is a negative correlation between the isotropy variable Q and the first principal component contribution ratio. Therefore, it is understood that, similar to the first principal component contribution ratio, the isotropy variable Q can be used as a surrogate characteristic indicating the degree of anisotropy of the forged material 22.

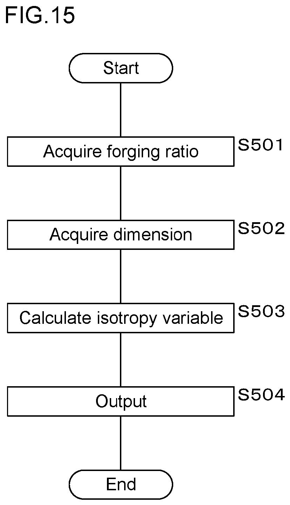

FIG. 15 is a flowchart illustrating a processing flow of the program 97 for calculating the isotropy variable Q. The control unit 11 acquires a forging ratio FR (step S501). Note that, in general, in a steel forging process, the forging ratio FR is an important management item and is recorded together with a lot number and the like.

The control unit 11 acquires dimensions (step S502). For example, the control unit 11 receives input of dimensions by the user. The user inputs, for example, dimensions of the dimensional-change measurement sample 41 that has been actually produced or is planned to be produced. The user may input dimensions of a virtual dimensional-change measurement sample 41.

The control unit 11 calculates the isotropy variable Q based on Equation (6) (step S503). The control unit 11 outputs the calculated isotropy variable Q. The control unit 11 ends the processing.

For example, the user inputs dimensions of a virtual sample in step S502 and checks an isotropy variable output in step S504. Based on a check result, the user determines whether to actually produce the dimensional-change measurement sample 41 and measure the dimensional change characteristics, or to proceed to manufacture of a product without performing actual measurement.

According to the present embodiment, by using the isotropy variable Q as a surrogate characteristic, it is possible to easily estimate the dimensional change characteristics of the forged material 22. That is, by using the isotropy variable Q, it is possible to realize an evaluation method for obtaining steel having stable quality.

Note that it is known that the characteristics of steel significantly vary depending on the amounts of elements. For example, by actually measuring dimensional change characteristics of an existing forged material 22, the graph described using FIG. 10 can be prepared. It is desirable to confirm, using the prepared graph, that there is a correlation between the isotropy variable Q and the dimensional change characteristics.

Thereafter, by finely adjusting the composition and the forging process and evaluating the isotropy variable Q, improved steel can be developed in a relatively short period of time. The forging process may be adjusted to obtain a desirable isotropy variable Q.

Recommended dimensions of components manufactured from the forged material 22 may be defined such that the isotropy variable Q falls within an allowable range.

A computer program can be deployed on a single computer or at one site, or distributed over a plurality of sites and loaded to be executed on a plurality of computers interconnected via a communication network.

Technical features (constituent elements) described in the respective embodiments can be combined with each other, and new technical features can be formed by such combinations.

The embodiments disclosed herein are to be considered illustrative in all respects and not restrictive. The scope of the invention is indicated by the claims rather than by the foregoing description, and all changes that come within the meaning and range equivalent to the claims are intended to be encompassed.

The independent claims and dependent claims described in the claims can be combined with each other in any and all combinations regardless of the citation format. Furthermore, the claims use a format in which a claim cites two or more other claims (multi-claim format). However, the invention is not limited thereto. A multi-claim that cites at least one multi-claim (multi-multi claim) may be used. Reference Signs List

| 10 | information processing apparatus |
| 11 | control unit |
| 12 | main storage device |
| 13 | auxiliary storage device |
| 14 | communication unit |
| 15 | display unit |
| 16 | input unit |
| 19 | reading unit |

| 21 | steel ingot |
|---|---|
| 22 | forged material |
| 31 | as-cast unit cell |
| 32 | post-forging unit cell |
| 40 | sample |
| 41 | dimensional-change measurement sample |
| 42 | mapping sample |
| 421 | mapping region |
| 96 | portable recording medium |
| 97 | program |
| 98 | semiconductor memory |

**Claims**

1. A method for evaluating steel, the method comprising:

   acquiring a forging ratio when a steel ingot is forged to obtain a forged material;
   acquiring dimensions of a rectangular parallelepiped-shaped sample, for the forged material, the sample having one side in an extension direction during forging; and
   calculating a variable related to isotropy of the sample based on the forging ratio and the dimensions.

2. The method for evaluating steel according to claim 1, wherein:

   the variable is an isotropy variable, and
   the isotropy variable is calculated by Equation (1):

$$Q = \frac{SL^2}{(ST \cdot SW) \cdot FR^3} \quad \cdots\cdots \quad (1)$$

   where Q is the isotropy variable, SL is a length of the side in the extension direction of the sample, ST is a length of a side of the sample perpendicular to the extension direction, SW is a length of another side of the sample perpendicular to the extension direction, and FR is the forging ratio.

3. The method for evaluating steel according to claim 2, comprising:

   actually measuring dimensional change characteristics of samples each taken from a respective one of a plurality of forged materials;
   calculating the isotropy variable for each sample; and
   outputting a graph using the isotropy variable on a first axis and the dimensional change characteristics on a second axis based on the isotropy variable and the dimensional change characteristics.

4. The method for evaluating steel according to claim 3, wherein the dimensional change characteristics are a sample standard deviation of dimensional change rates in respective side directions of the sample when the sample is subjected to heat treatment.

5. A program causing a computer to execute processes of:

   acquiring a forging ratio when a steel ingot in an as-cast state is forged;
   acquiring dimensions of a rectangular parallelepiped-shaped sample, for the steel ingot after forging, the sample having one side in an extension direction during forging; and
   calculating a variable relating to isotropy of the sample based on the forging ratio and the dimensions.

FIG.1

## FIG.2

FIG.3

10

Information Processing Apparatus

| 11 | 12 | 14 | 15 | 16 |
|---|---|---|---|---|
| Control Unit | Main Storage Device | Communication Unit | Display Unit | Input Unit |

98

13
Auxiliary Storage Device

19
Reading Unit

96

97

FIG.4

Sample 1-L

FIG.5

Sample 1-S

FIG.6

Sample 2-L

FIG.7

Sample 2-S

FIG.8

Sample 3-L

## FIG.9

Sample 4-L

FIG.10

FIG.11

1 mm

FIG.12

1 mm

FIG.13

FIG.14

FIG.15

```
        ┌─────────────┐
        │    Start    │
        └──────┬──────┘
               │
  ┌────────────────────────┐
  │  Acquire forging ratio │  S501
  └────────────┬───────────┘
               │
  ┌────────────────────────┐
  │    Acquire dimension   │  S502
  └────────────┬───────────┘
               │
  ┌────────────────────────┐
  │ Calculate isotropy variable │  S503
  └────────────┬───────────┘
               │
  ┌────────────────────────┐
  │        Output          │  S504
  └────────────┬───────────┘
               │
        ┌─────────────┐
        │     End     │
        └─────────────┘
```

## INTERNATIONAL SEARCH REPORT

| International application No. |
|---|
| **PCT/JP2024/033405** |

**A. CLASSIFICATION OF SUBJECT MATTER**

***G01N 33/20***(2019.01)i
FI:   G01N33/20 100

According to International Patent Classification (IPC) or to both national classification and IPC

**B. FIELDS SEARCHED**

Minimum documentation searched (classification system followed by classification symbols)

G01N33/20

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2009-132990 A (DAIDO STEEL CO., LTD.) 18 June 2009 (2009-06-18) paragraphs [0028], [0029], [0037], [0060], [0063], [0067]-[0069] | 1, 5 |
| A | | 2-4 |
| A | 前田雅人, 冷間ダイス鋼の熱処理による寸法変化の予測, 山陽特殊製鋼技報, 28 July 2017, vol. 24, no. 1, pp. 31-37, (MAEDA, Masato, Prediction of dimensional change during heat-treatment of cold work tool steel, Sanyo Technical Report) 3. Test Materials and Experiment Methods | 1-5 |
| A | 前田雅人, 冷間ダイス鋼の熱処理による寸法変化の予測, 材料とプロセス, 01 September 2016, vol. 29, no. 2, p. ROMBUNNO.251, (MAEDA, Masato, Prediction of dimensional change during heat-treatment of cold work tool steel, Current Advances in Materials and Processes) 2. Heat Treatment Dimensional Change Analysis Method | 1-5 |
| A | JP 2021-99257 A (DAIDO STEEL CO., LTD.) 01 July 2021 (2021-07-01) entire text, all drawings | 1-5 |

✓ Further documents are listed in the continuation of Box C.  ✓ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 November 2024** | **03 December 2024** |

| Name and mailing address of the ISA/JP | Authorized officer |
|---|---|
| **Japan Patent Office (ISA/JP) 3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT

| International application No. |
| --- |
| **PCT/JP2024/033405** |

### C.    DOCUMENTS CONSIDERED TO BE RELEVANT

| Category* | Citation of document, with indication, where appropriate, of the relevant passages | Relevant to claim No. |
| --- | --- | --- |
| A | JP 2003-65917 A (SUMITOMO ELECTRIC INDUSTRIES, LTD.) 05 March 2003 (2003-03-05)<br>    entire text, all drawings | 1-5 |
| A | 福元志保, 工具鋼におけるMs点予測モデルの妥当性の検討, 材料とプロセス, 01 September 2011, vol. 24, no. 2, p. ROMBUNNO.291, (FUKUMOTO, Shiho, Investigation of validity of the model for predicting Ms temperature for tool steel, Current Advances in Materials and Processes)<br>    entire text | 1-5 |
| P, A | WO 2023/182306 A1 (PROTERIAL, LTD.) 28 September 2023 (2023-09-28)<br>    entire text, all drawings | 1-5 |
| A | JP 2006-152356 A (HITACHI METALS LTD.) 15 June 2006 (2006-06-15)<br>    entire text, all drawings | 1-5 |
| A | JP 2006-169624 A (HITACHI METALS LTD.) 29 June 2006 (2006-06-29)<br>    entire text, all drawings | 1-5 |
| A | ROBERTS, G., CHAPTER 17 Troubleshooting: Manufacturing and Performance Problems In: Tool Steels, 5th Edition, ASM International, 1998, ISBN 978-1-6150-3201-3, pp. 325-343<br>    entire text, all drawings | 1-5 |
| A | 清水崇行, 冷間ダイス鋼の熱処理変寸の異方性に及ぼす晶出炭化物の影響, 電気製鋼, 29 November 2007, vol. 78, no. 4, pp. 289-298, (SHIMIZU, Takayuki, Influence of Coarse Carbide Volume on Anisotropy of Dimensional Change at Heat Treatment of Cold Work Die Steels, DENKI-SEIKO [ELECTRIC FURNACE STEEL])<br>    entire text, all drawings | 1-5 |
| A | US 2007/0185694 A1 (ELECTRICITE DE FRANCE-SERVICE NATIONAL) 09 August 2007 (2007-08-09)<br>    entire text, all drawings | 1-5 |

Form PCT/ISA/210 (second sheet) (July 2022)

INTERNATIONAL SEARCH REPORT
Information on patent family members

| International application No. |
| --- |
| **PCT/JP2024/033405** |

| Patent document cited in search report | | | Publication date (day/month/year) | Patent family member(s) | | | Publication date (day/month/year) |
| --- | --- | --- | --- | --- | --- | --- | --- |
| JP | 2009-132990 | A | 18 June 2009 | US 2009/0107587 A1 paragraphs [0071], [0072], [0124]-[0126], [0136], [0140]-[0145] EP 2055798 A1 KR 10-2009-0045093 A CN 101629267 A | | | |
| JP | 2021-99257 | A | 01 July 2021 | (Family: none) | | | |
| JP | 2003-65917 | A | 05 March 2003 | (Family: none) | | | |
| WO | 2023/182306 | A1 | 28 September 2023 | WO 2023/181435 A1 | | | |
| JP | 2006-152356 | A | 15 June 2006 | (Family: none) | | | |
| JP | 2006-169624 | A | 29 June 2006 | (Family: none) | | | |
| US | 2007/0185694 | A1 | 09 August 2007 | WO 2005/015167 A1 EP 1656546 A1 | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006152356 A
- JP 2006169624 A

**Non-patent literature cited in the description**

- **G.A.ROBERTS**. *Tool Steel 5th*, 1998, 338
- **F.RAPATZ**. *Die Edelstahl*, 1962, 945
- **TAKAYUKI SHIMIZU** ; **KOICHIRO INOUE**. Influence of Coarse Carbide Volume on Anisotropy of Dimensional Change at Heat Treatment of Cold Work Die steels. *Electric Steelmaking*, 2007, vol. 78 (4), 289
- **ISHIDA, K.** Calculation of the Effect of Alloying Elements on the Ms Temperature in Steels. *Journal of Alloys and Compounds*, 1995, vol. 220, 126-131